# EUROPEAN PATENT SPECIFICATION

(11) **EP 3 185 375 B1**
(45) Date of publication and mention of the grant of the patent: **08.09.2021**
(21) Application number: 15841320.3
(22) Date of filing: 14.07.2015
(51) Int. Cl.: H01T 19/04, A61L 9/22, H01T 23/00

(54) **DISCHARGE UNIT**
ENTLADUNGSEINHEIT
UNITÉ DE DÉCHARGE

(30) Priority: 19.09.2014 JP 2014191367
(43) Date of publication of application: 28.06.2017
(73) Proprietor: Daikin Industries, Ltd., Osaka-shi, Osaka 530-8323 (JP)
(72) Inventor: SUZUMURA, Kei, Osaka-shi, Osaka 530-8323 (JP); URABE, Daisuke, Osaka-shi, Osaka 530-8323 (JP); KAWABATA, Kazuyoshi, Osaka-shi, Osaka 530-8323 (JP)
(74) Representative: Goddar, Heinz J.
(86) International application number: PCT/JP2015/003543
(87) International publication number: WO 2016/042694

(56) References cited:
- EP-A1- 2 637 269
- JP-A- H0 330 847
- JP-A- H04 310 251
- JP-A- 2005 135 894
- JP-A- 2005 135 894
- JP-A- 2011 018 616
- JP-A- 2013 152 910
- JP-A- 2013 218 785
- JP-A- 2014 119 186
- US-A1- 2008 316 672

## Description

### TECHNICAL FIELD

The present invention relates to a discharge unit.

### BACKGROUND ART

Discharge units which cause a discharge between a discharge electrode and a counter electrode have been known. For example, a discharge unit disclosed in Patent Document 1 is mounted in an air conditioner. The discharge unit includes a discharge electrode having a discharge needle, a counter electrode facing the tip of the discharge needle, and a voltage supply section which applies a potential difference between these electrodes. When a voltage is supplied to the discharge electrode from the voltage supply section, a streamer discharge is generated from the tip of the discharge needle toward the counter electrode. Due to this streamer discharge, active species (e.g., electrons, ions, radicals and ozone) are generated in the air. These active species decompose harmful or odor components in the air.

EP 2 637 269 A1 and JP 2011 018616 A disclose a discharge unit comprising a casing in which an air passage is formed to allow air to pass therethrough, a discharge electrode located in the air passage, a counter electrode located in the air passage, a voltage supply section configured to give a potential difference between the discharge electrode and the counter electrode and an insulating member.

US 2008/316672 A1 discloses variations to the geometry of the support structure to decrease the creepage.

JP 2013 152910 A discloses that different forms of electrodes can be used.

JP 2005 135894 A discloses supports of electrodes and the phenomenon of creepage and how to reduce it.

### CITATION LIST

### PATENT DOCUMENT

Patent Document 1: Japanese Unexamined Patent Publication No. 2014-119186

### SUMMARY OF THE INVENTION

### TECHNICAL PROBLEM

The inventors of the present application have reached a structure of the above-described discharge unit in which the discharge electrode and the counter electrode are supported by an insulating member. This structure allows the discharge electrode and the counter electrode to have a uniform relative distance between each other, and allows insulation between the discharge electrode and the counter electrode.

The inventors of the present application have also reached a structure in which a recess is formed in the insulating member to increase the creepage distance between the discharge electrode and the counter electrode. This makes it possible to prevent a leakage current from the discharge electrode to the counter electrode, keep a potential difference between the two electrodes, and hence provide a stable discharge.

However, the recess formed in the insulating member may result in having dust or other particles contained in the air adhered to the inner wall of the recess. Such dust or other particles which adhere to the wall surface may increase a leakage current on the surface of the insulating member and cause a problem that the insulating member cannot maintain its desired insulation resistance.

In view of the foregoing, it is therefore an object of the present invention to prevent dust or other particles from adhering to an inner wall of a recess of an insulating member.

### SOLUTION TO THE PROBLEM

A first aspect of the present disclosure is directed to a discharge unit according to claim 1.

According to the first aspect of the present disclosure, the counter electrode (60) is supported on the periphery of the base (44) formed in the insulating member (41), and the discharge electrode (70) is supported on the support (47) of the insulating member (41). As a result, a relative positional relationship between the discharge electrode (70) and the counter electrode (60) is determined, and the discharge electrode (70) and the counter electrode (60) are insulated from each other. The base (44) is provided with a recess (46), from the bottom of which the support (47) protrudes to the outside of the open end of the recess (46). The support (47) supports the discharge electrode (70). Thus, the discharge electrode (70) and the counter electrode (60) are connected to each other by way of the creepage surface including, e.g., the outer wall surface of the support (47), the bottom of the recess (46), and the inner wall surface of the recess (46). As a result, the creepage distance between the discharge electrode (70) and the counter electrode (60) is increased, which leads to an increase in the insulation resistance between the discharge electrode (70) and the counter electrode (60).

The recess (46) of the insulating member (41) extends in a direction intersecting with the flow of the air passing through the air passage (27). Thus, the amount of air which flows through the air passage (27) and comes into the recess (46) may be reduced, which in turn may reduce the amount of dust in the air adhering to the inner wall surface of the recess (46).

According to the first aspect, the base (44) extends in a direction intersecting with the flow of air, and the recess (46), too, extends in the direction intersecting with the flow of air. In this structure, the distance in which air flowing through the air passage (27) passes through the base (44) is shortened and the dust or other particles in the air may be prevented from entering the recess (46). As a result, the amount of dust in the air adhering to the inner wall surface of the recess (46) may be reduced.

A second aspect of the present disclosure is an embodiment of the first aspect of the present disclosure. In the second aspect, the support (47) is located at a central position of the recess (46).

According to the second aspect, the support (47) is located at a central position of the recess (46). This structure makes a distance between the support (47) and the inner wall surface of the recess (46) relatively uniform. As a result, the creepage distance between the discharge electrode (70) and the counter electrode (60) becomes uniform, too, which leads to an increase in the insulation resistance of the insulating member (41).

A third aspect of the present disclosure is an embodiment of the first aspect of the present disclosure. In the third aspect, the discharge electrode (70) includes an electrode support plate (71) extending along the base (44) and supported by the support (47), and elongated discharge needles (73, 74) arranged at longitudinal side edges of the electrode support plate (71) and protruding from the longitudinal side edges. The counter electrode (60) includes counter plates (61, 62) which extend along a longitudinal direction of the base (44) and to which tips of the discharge needles (73, 74) are opposed. Gaps (65, 66) laterally extending along the longitudinal direction of the base (44) are formed between the base (44) and the counter plates (61, 62).

According to the third aspect, a plurality of thin discharge needles (73, 74) are arranged at the side edges of the electrode support plate (71), and the discharge needles (73, 74) protrude from the side edges. The counter electrode (60) includes counter plates (61, 62) to which tips of the discharge needles (73, 74) are opposed. Thus, a discharge occurs from the tips of the discharge needles (73, 74) to the counter plates (61, 62).

The counter plates (61, 62) extend along the longitudinal direction of the base (44). This structure may lead to a relatively short distance between the discharge electrode (70) and each counter plate (61, 62), which in turn may shorten the creepage distance between the discharge electrode (70) and each counter plate (61, 62). However, the provision of the gaps (65, 66) elongated in the left-and-right direction between the base (44) and each counter plate (61, 62) may increase the creepage distance between the discharge electrode (70) and each counter plate (61, 62).

A fourth aspect of the present disclosure is an embodiment of the third aspect of the present disclosure. In the fourth aspect, the plurality of discharge needles (73, 74) of the discharge electrode (70) are arranged along the respective side edges, in a width direction, of the electrode support plate (71). The counter plates (61, 62) of the counter electrode (60) are located on respective sides, in the width direction, of the base (44) to correspond to rows of the discharge needles (73, 74). The gap (65, 66) is individually formed between the base (44) and a corresponding one of the counter plates (61, 62).

According to the fourth aspect, the plurality of discharge needles (73, 74) are arranged along the respective side edges, in the width direction, of the electrode support plate (71). As a result, a large number of discharge needles (73, 74) may be provided, and the amount of active species generated by the discharge may also be increased.

Individually providing the gap (65, 66) between the base (44) and a corresponding one of the counter plates (61, 62) may prevent a short creepage distance between the discharge electrode (70) and the counter plates (61, 62).

### ADVANTAGES OF THE INVENTION

According to one or more aspects of the present disclosure, the recess (46) formed in the insulating member (41) is recessed in the direction intersecting with the flow of air. Thus, the dust in the air may be prevented from entering the recess (46). As a result, it is possible to prevent the dust from adhering, e.g., to the inner wall of the recess (46) and hence to prevent reduction in the insulation resistance of the insulating member (41). This may ensure a sufficient potential difference between the discharge electrode (70) and the counter electrode (60) and achieve desired discharges.

According to the second aspect, a uniform creepage distance from the support (47) to the inner wall surface of the recess (46) is achieved, which may reduce the risk that part of the creepage distance is significantly shortened and a leakage current increases. As a result, a sufficient insulation resistance of the insulating member (41) may be ensured.

According to the first aspect, the base (44) and the recess (46) extend in the direction intersecting with the flow of air. Thus, the distance traveled by the air passing through the recess (46) may be minimized. As a result, it is possible to further reduce the amount of dust in the air which adheres, e.g., to the inner wall of the recess (46).

According to the third and fourth aspects, the provision of the gaps (65, 66) between the base (44) and each counter plate (61, 62) may further increase the creepage distance between the discharge electrode (70) and each counter plate (61, 62). In addition, according to the fifth aspect, the electrode support plate (71) may support a large number of discharge needles (73, 74) on the electrode support plate (71), which may lead to a further increase in the amount of active species generated by a discharge.

### BRIEF DESCRIPTION OF THE DRAWINGS

FIG. 1 is a schematic diagram illustrating an air conditioner of an embodiment.
FIG. 2 is a perspective view of a casing of a discharge unit, when viewed from the front side.
FIG. 3 is a perspective view of the casing of the discharge unit, when viewed from the rear side.
FIG. 4 is a perspective view of an internal structure of the discharge unit.
FIG. 5 is an assembly diagram of a discharge process section and its peripheral devices.
FIG. 6 is a plan view of the discharge process section and its peripheral devices for illustrating a state in which the discharge electrode and a stabilizer are removed from the discharge process section.
FIG. 7 is a plan view of the discharge process section and its peripheral devices for illustrating a state in which the stabilizer is removed from the discharge process section.
FIG. 8 is a plan view of the discharge process section and its peripheral devices.
FIG. 9 is a cross-sectional view of FIG. 8 taken along the plane IX-IX.
FIG. 10 is a perspective view of the stabilizer.
FIG. 11 is a diagram when viewed along the arrow XI of FIG. 8.
FIG. 12 is a horizontal cross-sectional view illustrating the insulating member and its peripheral devices.
FIG. 13 is a cross-sectional view of FIG. 8 taken along the plane XIII-XIII.

### DESCRIPTION OF EMBODIMENTS

Embodiments of the present invention will be described in detail below, with reference to the drawings. The following embodiments are merely exemplary ones in nature, and are not intended to limit the scope, applications, or use of the invention.

A discharge unit (20) of the present invention is mounted in an air conditioner (10). The air conditioner (10) adjusts the temperature of air in an indoor space (S).

### <Configuration for Air Conditioner>

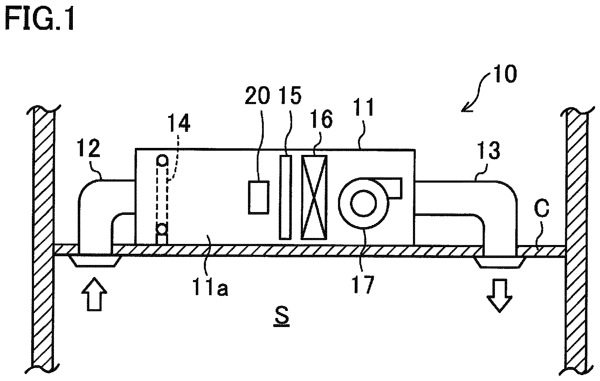

As illustrated in FIG 1, the air conditioner (10) is installed on the back side of a ceiling (C). The air conditioner (10) has an air conditioner casing (11) having a horizontally-oriented box-like shape. A room-air duct (12) is connected to one of the longitudinal side surfaces of the air-conditioner casing (11). An air-supply duct (13) is connected to the other longitudinal side surface of the air-conditioner casing (11). An air passage (11a) is formed in the interior of the air-conditioner casing (11). The room-air duct (12) has an intake end communicating with the indoor space (S) and an outlet end communicating with the air passage (11a). The air-supply duct (13) has an intake end communicating with the air passage (11a) and an outlet end communicating with the indoor space (S).

The air passage (11a) is provided with a prefilter (14), a discharge unit (20), a catalyst filter (15), a heat exchanger (16), and a fan (17) sequentially arranged from the upstream to downstream side of the air flow (i.e., from the room-air duct (12) to the air-supply duct (13)). The prefilter (14) catches relatively-large dust in the air. In the discharge unit (20), active species are generated by a discharge, and these active species decompose harmful or odor components in the air.

The catalyst filter (15) is comprised, for example, of a base with a honeycomb structure which carries a catalyst on its surface. A manganese-based catalyst or a noble metal-based catalyst is used as the catalyst. The catalyst filter (15) further activates the active species generated by the discharge to promote decomposition of the harmful or odor components in the air. The catalyst filter (15) carries an adsorbent (e.g., activated carbon) which adsorbs the harmful or odor components in the air.

The heat exchanger (16) heats and cools the air flowing through the air passage (11a). Specifically, the heat exchanger (16) is connected to a refrigerant circuit (not shown). The refrigerant circuit is filled with a refrigerant which circulates to perform a refrigeration cycle. The heat exchanger (16) functions as an evaporator which cools the air with a low-pressure refrigerant flowing in the heat exchanger. The heat exchanger also functions as a condenser which heats the air with a high-pressure refrigerant flowing in the heat exchanger. The fan (17) transfers the air in the air passage (11a).

### <Configuration for Discharge Unit>

The discharge unit (20) is configured as a streamer discharge unit. That is, the discharge unit (20) performs a streamer discharge, thereby generating low-temperature plasma, which generates highly reactive active species (e.g., high-speed electrons, ions, radicals and ozone) in the air. The discharge unit (20) includes a casing (21), a voltage supply section (30) housed in the casing (21), and a discharge process section (40) housed in the casing (21).

### [Casing]

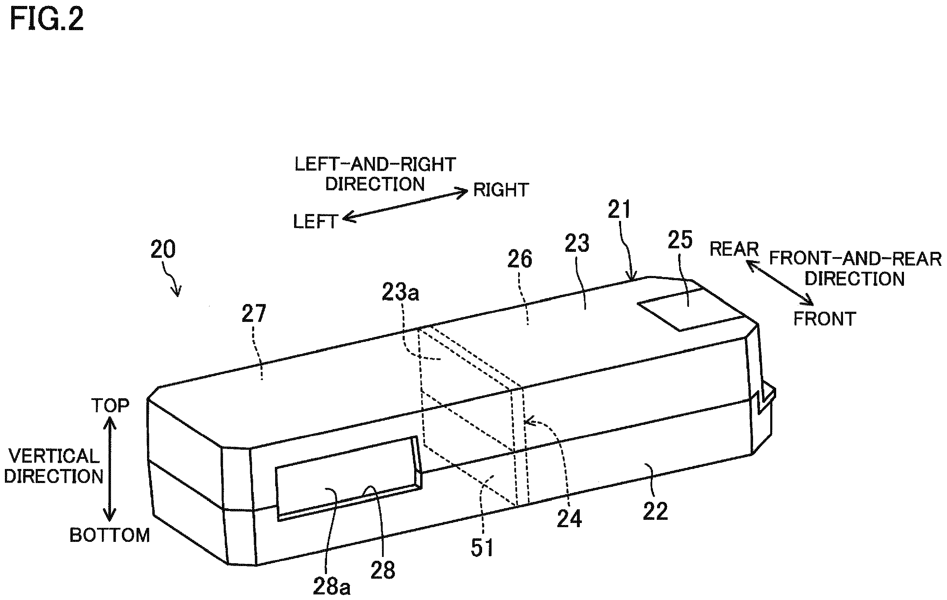

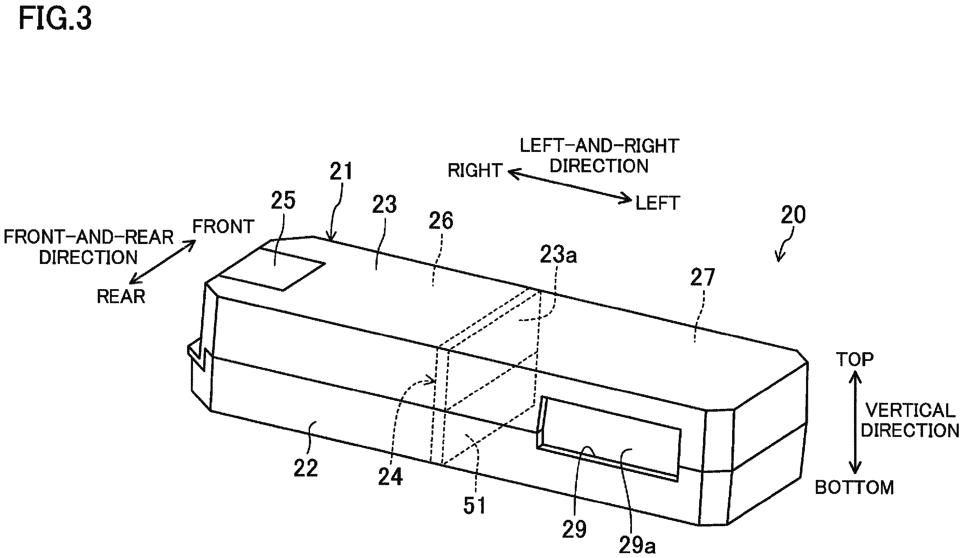

As illustrated in FIG. 2 and FIG. 3, the casing (21) has a horizontally-oriented, generally parallelepiped box-like shape. The casing (21) is made of an insulating resin material. The casing (21) includes a lower case (22) and an upper case (23) attached to the upper side of the lower case (22). A partition (24) is provided in the casing (21) at a longitudinal middle portion (i.e., a middle portion in the left-and-right direction) of the casing (21). The partition (24) divides the interior of the casing (21) into two spaces (i.e., left and right spaces). The right space constitutes a housing chamber (26), and the left space constitutes a processing chamber (27) (i.e., an air passage).

The partition (24) is comprised of an upper partition wall (23a) and a lower partition wall (51). The upper partition wall (23a) is an integrally-formed wall in the upper case (23). The lower partition wall (51) is formed integrally with an insulating member (41), which will be described later. The upper partition wall (23a) and the lower partition wall (51) of the partition (24) are arranged one above the other such that the lower surface of the upper partition wall (23a) and the upper surface of the lower partition wall (51) are adjacent to each other.

As illustrated in FIG. 2, the front surface of the casing (21) is provided with a first air hole (28) (i.e., an inlet hole). The first air hole (28) is positioned closer to the left side of the casing (21) so that the hole may communicate with the processing chamber (27). A first shielding plate (28a) is provided behind the first air hole (28). The first shielding plate (28a) has a rectangular shape generally larger in size than the opening area of the first air hole (28). The first shielding plate (28a) serves as a shielding member which avoids exposure of the discharge process section (40) (namely, discharge needles (73, 74) of the discharge electrode (70), etc.) to the outside of the casing (21). A gap (i.e., an air inflow path) is formed between the edge of the first air hole (28) and the first shielding plate (28a). The air which has flowed in through the first air hole (28) passes through this gap to flow into the interior of the processing chamber (27).

As illustrated in FIG. 3, the rear surface of the casing (21) is provided with a second air hole (29). The second air hole (29) is positioned closer to the left side of the casing (21) so that the hole may communicate with the processing chamber (27). A second shielding plate (29a) is provided behind the second air hole (29). The second shielding plate (29a) has a rectangular shape generally larger in size than the opening area of the second air hole (29). The second shielding plate (29a) serves as a shielding member which avoids exposure of the discharge process section (40) (namely, the discharge needles (73, 74) of the discharge electrode (70), etc.) to the outside of the casing (21). A gap (i.e., an air intake path) is formed between the edge of the second air hole (29) and the second shielding plate (29a). The air in the processing chamber (27) passes through this gap to flow out of the casing (21).

The first shielding plate (28a) and the second shielding plate (29a) are integrally formed with the casing (21). The first shielding plate (28a) and the second shielding plate (29a) are made of a resin material with a higher flame-resistance than the material forming the casing (21).

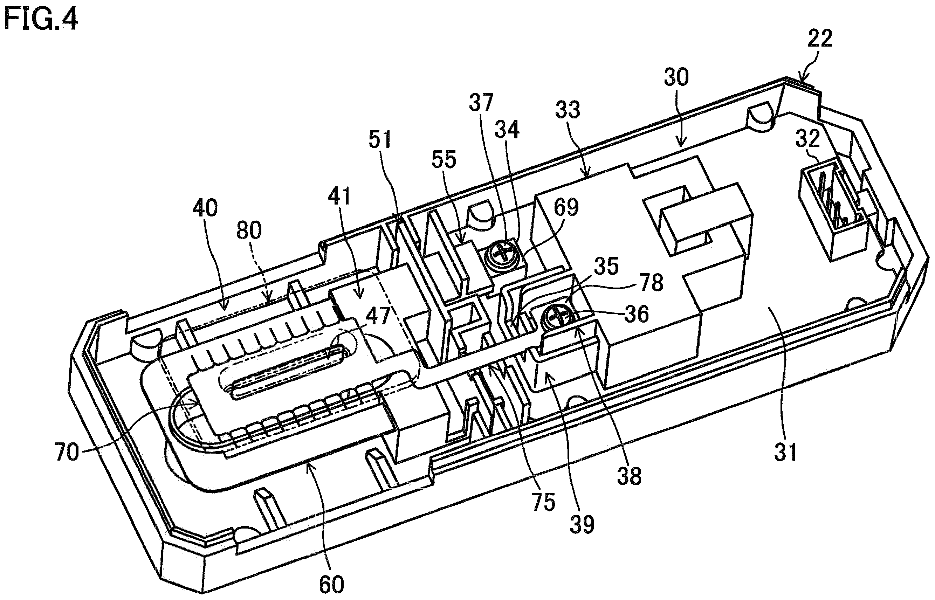

As illustrated in FIG. 2 and FIG. 3, a slidable cover (25) is provided on the right end of the upper case (23) at a middle portion in the front-and-rear direction. The slidable cover (25) may be attached to, or detached from, the body of the casing (21). If the slidable cover (25) is detached, a connector (32) (see FIG. 4) of the voltage supply section (30) is exposed to the outside of the casing (21).

### [Voltage Supply Section]

As illustrated in FIG. 4, the voltage supply section (30) is located in the housing chamber (26). The voltage supply section (30) is configured to supply source voltage supplied from an external power source to the discharge process section (40). The voltage supply section (30) has a substrate (31), a connector (32), a power transformer (33), and an earth terminal (34). The substrate (31) is arranged near the bottom of the housing chamber (26). The substrate (31) has a plate-like shape elongated in the right-and-left direction and is arranged to lie across the entire region of the housing chamber (26).

The connector (32) is arranged on the upper surface of a right end portion of the substrate (31). The connector (32) is exposed to the outside of the casing (21) when the above-described slidable cover (25) is detached. A wire electrically connected to the external power source is connected to the connector (32).

The power transformer (33) is arranged on the upper surface of the substrate (31) at a location closer to the right side of the substrate (31). The power transformer (33) is configured to boost the voltage supplied to the power transformer (33) via the connector (32). A feed terminal (35) is provided at a left end portion of the power transformer (33). A feed plate (75) of the discharge electrode (70) is fixed to the feed terminal (35) with a fastening member (i.e., a screw (36)).

The earth terminal (34) is arranged on the upper surface of the substrate (31) at a location closer to the left end and the rear end of the substrate (31). An earth plate (68) of the counter electrode (60) is fixed to the earth terminal (34) with a fastening member (i.e., a screw (37)).

The feed terminal (35) is located at a relatively higher position than the earth terminal (34). In other words, the feed terminal (35) and the earth terminal (34) are at different positions in the vertical direction. The feed terminal (35) is located closer to the processing chamber (27) than the earth terminal (34) is. In other words, the feed terminal (35) and the earth terminal (34) are at different positions in the left-and-right direction (i.e., in the longitudinal direction of the discharge electrode (70) or the counter electrode (60)). Further, the feed terminal (35) is located closer to the front side than the earth terminal (34) is. In other words, the feed terminal (35) and the earth terminal (34) are at different positions in the front-and-rear direction. This structure increases the distance between the feed terminal (35) and the earth terminal (34) and thus contributes to an increase in a creepage distance between the feed terminal (35) and the earth terminal (34).

The power transformer (33) has an inner wall portion (38) which surrounds the feed terminal (35). The inner wall portion (38) is made of an insulating resin material of which the upper side and the left side are open. The inner wall portion (38) has a cross-section having a square U shape whose left side is open (i.e., an inverted C shape).

The inner wall portion (38) is surrounded by an outer wall portion (39). The outer wall portion (39) is made of an insulating resin material of which the upper side and the right side are open. The outer wall portion (39) has a cross-section having a square U shape whose right side is open (i.e., a C shape). There is a gap between the inner wall portion (38) and the outer wall portion (39) along the entire periphery.

The provision of the inner wall portion (38) and the outer wall portion (39) surrounding the feed terminal (35) contributes to a further increase in the creepage distance between the feed terminal (35) and the earth terminal (34).

### [Discharge Process Section]

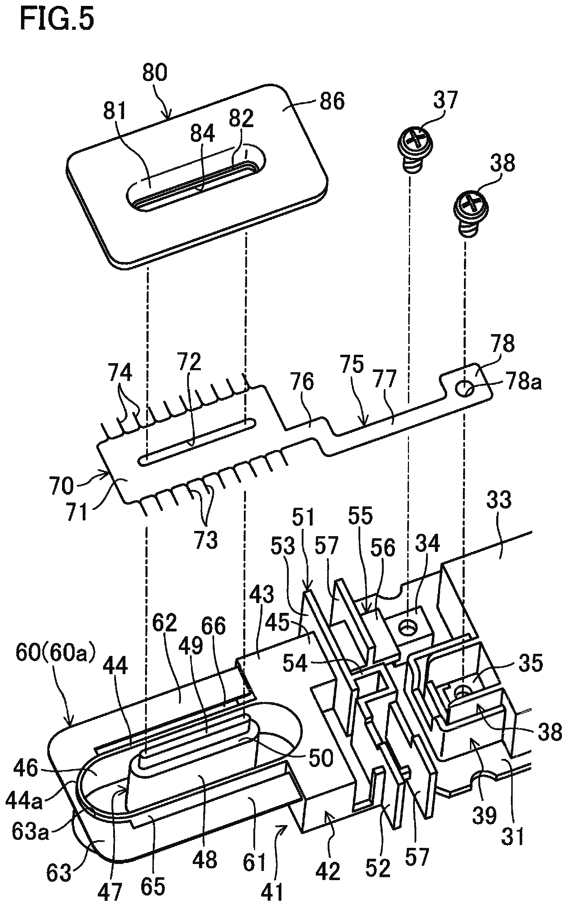

As illustrated in FIG 4 and FIG. 5, the discharge process section (40) is generally located in the processing chamber (27). The discharge process section (40) is configured to generate a streamer discharge to purify air. The discharge process section (40) includes an insulating member (41), a counter electrode (60), a discharge electrode (70), and a stabilizer (80).

The insulating member (41) is made of an insulating resin material and serves as a support member which supports the discharge electrode (70) and the counter electrode (60) while insulating these electrodes from each other. The counter electrode (60) and the discharge electrode (70) are made of a conductive metal material. The counter electrode (60) is electrically connected to an earth connector (69) and is grounded. The discharge electrode (70) is electrically connected to the voltage supply section (30), and receives a high voltage (e.g., 7.0 kV). When a voltage is supplied to the discharge electrode (70) from the voltage supply section (30), a streamer discharge is generated between the two electrodes (60, 70). The stabilizer (80) is made of a conductive resin material, and has the same electric potential as the discharge electrode (70). The stabilizer (80) serves as a conductive member (i.e., a fixing member) for creating a stable electric field near the discharge electrode (70).

### [Insulating Member]

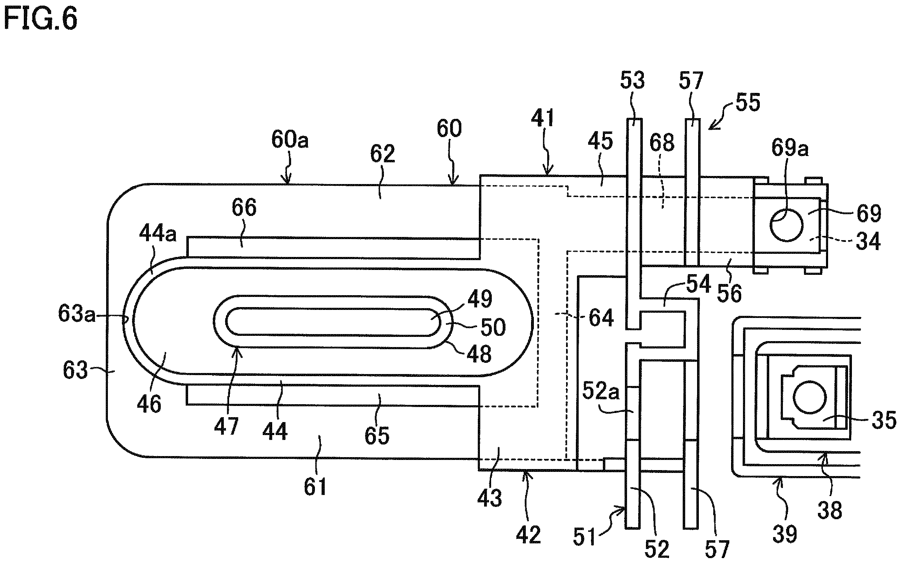

As illustrated in FIG 4, the insulating member (41) is placed on the bottom of the lower case (22). As is also illustrated in FIG 5, FIG. 6 and FIG. 9, the insulating member (41) includes an embedding portion (42), a base (44), a support (47), a lower partition wall (51), and a creepage distance increase portion (55).

The embedding portion (42) is arranged in the processing chamber (27) at a location on the left side of the lower partition wall (51). The embedding portion (42) has a body (43) and a connecting portion (45). The body (43) has a rectangular parallelepiped shape extending between the front edge and the rear edge of the lower case (22). The connecting portion (45) is formed continuously between a rear end portion of the right side surface of the body (43) and the lower partition wall (51).

The base (44) protrudes to the left from a middle portion, in the front-and-rear direction, of the left side surface of the body (43). The base (44) includes an arc portion (44a) at its end which has an arc-shaped cross section. The insulating member (41) is provided with an oblong groove (46) (i.e., a recess) extending from the base (44) to a middle portion of the body (43). The oblong groove (46) is a cylindrical hole having an oblong shape elongated in the left-and-right direction, with a closed bottom and an open top.

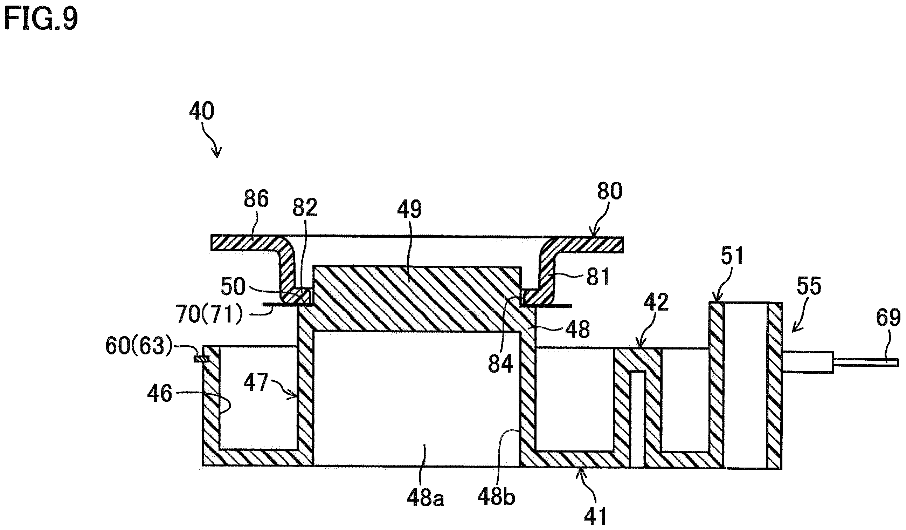

The support (47) is located at a middle portion, in the left-and-right direction and the front-and-rear direction, of the oblong groove (46). The support (47) includes a support body (48) and a projection (49) (i.e., a fitted portion) which projects upward from the support body (48). The support body (48) has a columnar shape having an oblong transverse cross-section elongated in the left-and-right direction. The support body (48) has a hollow (48a) therein which extends upward from the lower end of the support body (48) (see FIG. 9). The support body (48) has an inner periphery surface (48b) which forms an oblong shape elongated in the left-and-right direction to surround the hollow (48a).

The projection (49) is located at a middle portion, in the left-and-right direction and the front-and-rear direction, of the support body (48). Similarly to the support body (48), the projection (49) has a columnar shape having an oblong cross-section elongated in the left-and-right direction. The projection (49) has a height, a width in the left-and-right direction, and a thickness in the front-and-rear direction, all of which are less than those of the support body (48). Thus, a ring-shaped mounting surface (50) that is oblong in the left-and-right direction is formed on the upper end surface of the support body (48) so as to surround the projection (49). The mounting surface (50) is approximately level and flat. The support (47) supports the discharge electrode (70) and the stabilizer (80) which will be described in detail later.

The lower partition wall (51) extends between the front edge and the rear edge of the lower case (22). The lower partition wall (51) includes a first lateral wall (52) positioned closer to the front side of the lower case (22), a second lateral wall (53) positioned closer to the rear side of the lower case (22), and a protruding wall (54) which protrudes to the right from between the lateral walls (52, 53). The first lateral wall (52) and the second lateral wall (53) have a plate-like shape extending in the front-and-rear direction. The protruding wall (54) has a cross-section having a square U shape whose left side is open (i.e., an inverted C shape) and is continuous with a rear portion of the first lateral wall (52) and a front portion of the second lateral wall (53). A vertically-oriented gap is formed between the first lateral wall (52) and the second lateral wall (53), and this gap communicates with the space surrounded by the protruding wall (54).

The creepage distance increase portion (55) is configured of a plurality of horizontal plates (56) and a plurality of vertical plates (57) assembled to intersect with each other. Some of these plates are continuous with the lower partition wall (51). As such, the lower partition wall (51) and the creepage distance increase portion (55) form a so-called labyrinth structure, in which a plurality of insulating plates having vertical or horizontal surfaces are complicatedly assembled to one another. As a result, the creepage distance between the discharge electrode (70) and the counter electrode (60) is increased.

### [Counter Electrode]

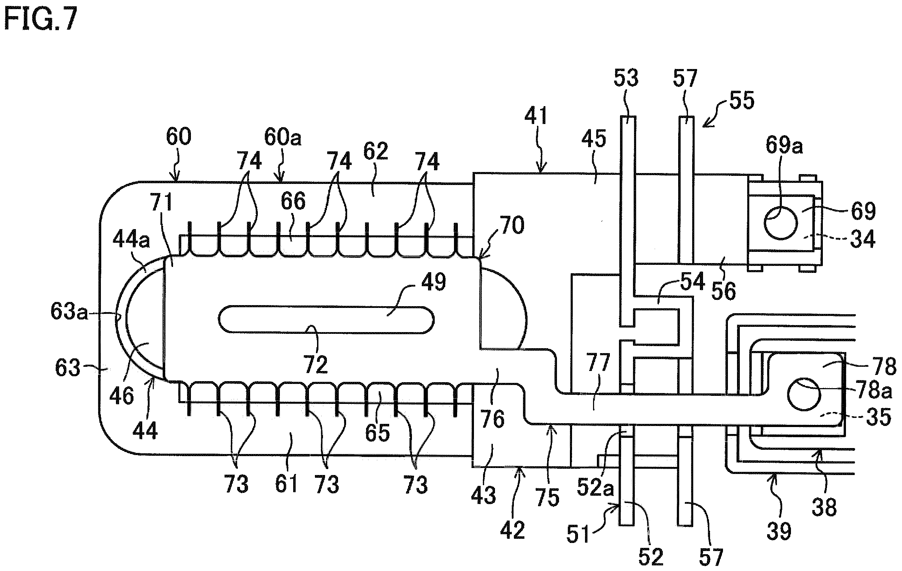

As illustrated in FIG. 4 to FIG. 7 and FIG. 9, the counter electrode (60) is integrally formed with the insulating member (41). Specifically, the counter electrode (60) and the insulating member (41) are configured as an integrally-formed unit formed by insert molding. The counter electrode (60) has a flat plate-like shape so that the entire portion thereof may lie on the same plane (i.e., on a horizontal plane). The counter electrode (60) includes a rectangular frame-like counter electrode body (60a) and an earth plate (68) extending to the right from a rear portion of the right side of the counter electrode body (60a).

The counter electrode body (60a) is comprised of a first counter plate (61), a second counter plate (62), a first connecting plate (63), and a second connecting plate (64) which are assembled to one another. The first counter plate (61) is positioned closer to the front of the counter electrode body (60a) and extends in the left-and-right direction. The second counter plate (62) is positioned closer to the rear of the counter electrode body (60a) and extends in the left-and-right direction. A first gap (65) having a rectangular shape elongated in the left-and-right direction is formed between the first counter plate (61) and the front side of the base (44). A second gap (66) having a rectangular shape elongated in the left-and-right direction is formed between the second counter plate (62) and the rear side of the base (44).

The first connecting plate (63) is positioned closer to the left of the counter electrode body (60a) and extends in the front-and-rear direction. The first connecting plate (63) connects the left end of the first counter plate (61) with the left end of the second counter plate (62). Formed at an inner periphery (i.e., the right side) of the first connecting plate (63) is an arc groove (63a) to which the arc portion (44a) of the base (44) is fitted. The second connecting plate (64) is positioned closer to the right of the counter electrode body (60a) and extends in the front-and-rear direction. The second connecting plate (64) connects the right end of the first counter plate (61) with the right end of the second counter plate (62). The second connecting plate (64) is embedded in an upper portion of the base (43).

Almost the entire portion of the earth plate (68) is embedded in the embedding portion (42) of the insulating member (41) and the horizontal plates (56). The earth plate (68) has a tip end which protrudes further to the right from the horizontal plate (56) and is exposed to the outside of the insulating member (41). The tip end of the earth plate (68) serves as the earth connector (69). The earth connector (69) has an approximately square plate-like shape and is provided with an insertion hole (69a) through the center of which a screw (37) is inserted. The earth connector (69) is placed to be layered on the upper surface of the earth terminal (34) and is connected to the earth terminal (34) with the screw (37).

### [Discharge Electrode]

As illustrated in FIG. 4, FIG. 5, FIG. 7 to FIG. 9, and FIG. 11, the discharge electrode (70) is arranged above the insulating member (41). The discharge electrode (70) has a thin plate-like shape so that the entire portion thereof may lie on the same plane (i.e., on a horizontal plane). The discharge electrode (70) is much thinner than the counter electrode (60). The discharge electrode (70) includes an electrode support plate (71), a plurality of discharge needles (73, 74) supported on the side edges of the electrode support plate (71), and a feed plate (75) protruding to the right from a front end portion of the right side of the electrode support plate (71).

The electrode support plate (71) is arranged above the base (44). The electrode support plate (71) extends in the left-and-right direction along the base (44). A positioning hole (72) (i.e., an opening), into which the projection (49) of the support (47) is fitted, is formed at the center of the electrode support plate (71) (i.e., a middle portion in the longitudinal direction and width direction of the electrode support plate (71)). The positioning hole (72) has an oblong shape elongated in the left-and-right direction to correspond to the profile of the projection (49). Once the projection (49) is fitted in the positioning hole (72), the electrode support plate (71) is brought on the mounting surface (50). As a result, the flatness of the electrode support plate (71) is maintained. In other words, the electrode support plate (71) is kept flat by being supported on the mounting surface (50). A relative positional relationship between the discharge electrode (70) and the insulating member (41) is also determined, which in turn determines a relative positional relationship between the discharge electrode (70) and the counter electrode (60).

In the state in which the projection (49) is fitted in the positioning hole (72), a slight gap (not shown) is left between longitudinal end portions (i.e., left and right end portions) of the projection (49) and the inner periphery of the positioning hole (72). That is, the positioning hole (72) has a longer maximum longitudinal length than the projection (49). This slight gap allows the projection (49) to thermally expand (i.e., extend under heat) in its longitudinal direction in the positioning hole (72). Thus, stress may be prevented from being concentrated at both longitudinal ends of the inner periphery of the positioning hole (72) when the thermal expansion of the projection (49) occurs.

The plurality of first discharge needles (73), each in the form of a thin needle or rod, are supported on the front edge of the electrode support plate (71). The plurality of first discharge needles (73) are arranged at regular intervals along the front edge of the electrode support plate (71) and extend horizontally straight forward from the electrode support plate (71). The first discharge needles (73) are arranged parallel to one another. The plurality of second discharge needles (74), each in the form of a thin needle or rod, are supported on the rear edge of the electrode support plate (71). The plurality of second discharge needles (74) are arranged at regular intervals along the rear edge of the electrode support plate (71) and extend horizontally straight rearward. The second discharge needles (74) are arranged parallel to one another. The electrode support plate (71) extends in a direction in which the plurality of discharge needles (73, 74) are arranged. As such, a large number of discharge needles (73, 74) may be provided at the front and rear side edges of the electrode support plate (71).

The discharge electrode (70) of the present embodiment is provided with ten first discharge needles (73) and ten second discharge needles (74). The number of these discharge needles (73, 74) is merely an example. The first discharge needle (73) and the second discharge needle (74) are generally coaxial with each other in the front-and-rear direction. The first discharge needle (73) and the second discharge needle (74) may be misaligned with each other in the left-and-right direction.

The first discharge needles (73) are parallel to the first counter plate (61). The second discharge needles (74) are parallel to the second counter plate (62). A lower portion of the tip of each first discharge needle (73) faces the first counter plate (61), and a lower portion of the tip of the second discharge needle (74) faces the second counter plate (62).

The feed plate (75) includes, from left to right, a first feed portion (76), a second feed portion (77), and a feed connecting portion (78). The first feed portion (76) protrudes to the right from a front edge portion of the right side of the electrode support plate (71). The second feed portion (77) is connected to a front edge portion of the tip of the first feed portion (76) and protrudes to the right. A portion of the second feed portion (77) is supported on the bottom of a recessed groove (52a) of the first lateral wall (52). The tip end of the second feed portion (77) serves as the feed connecting portion (78). The feed connecting portion (78) has an approximately square plate-like shape and is provided with an insertion hole (78a) through the center of which a screw (36) is inserted. The feed connecting portion (78) is placed to be layered on the upper surface of the feed terminal (35) and is connected to the feed terminal (35) with the screw (36).

### [Stabilizer]

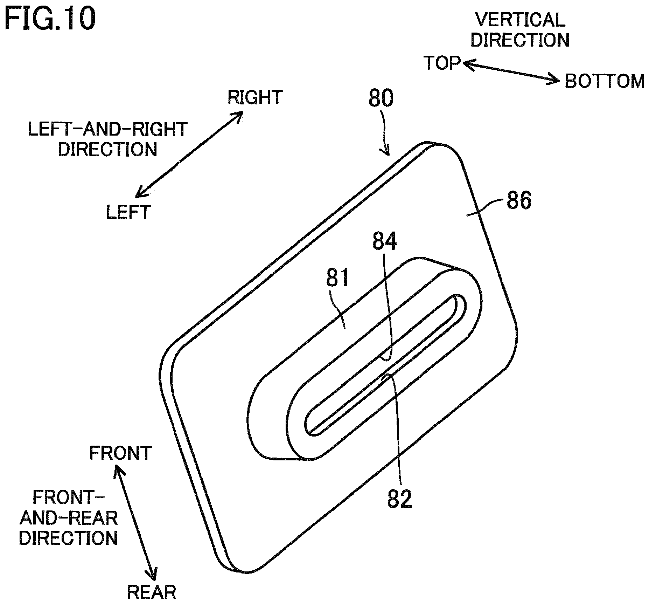

The stabilizer (80) is arranged above the support (47) and the discharge electrode (70). As illustrated in FIG. 5 and FIG. 8 to FIG. 11, the stabilizer (80) includes a cylindrical wall portion (81) and a flanged portion (86) projecting out from an upper end portion of the cylindrical wall portion (81) to all the directions.

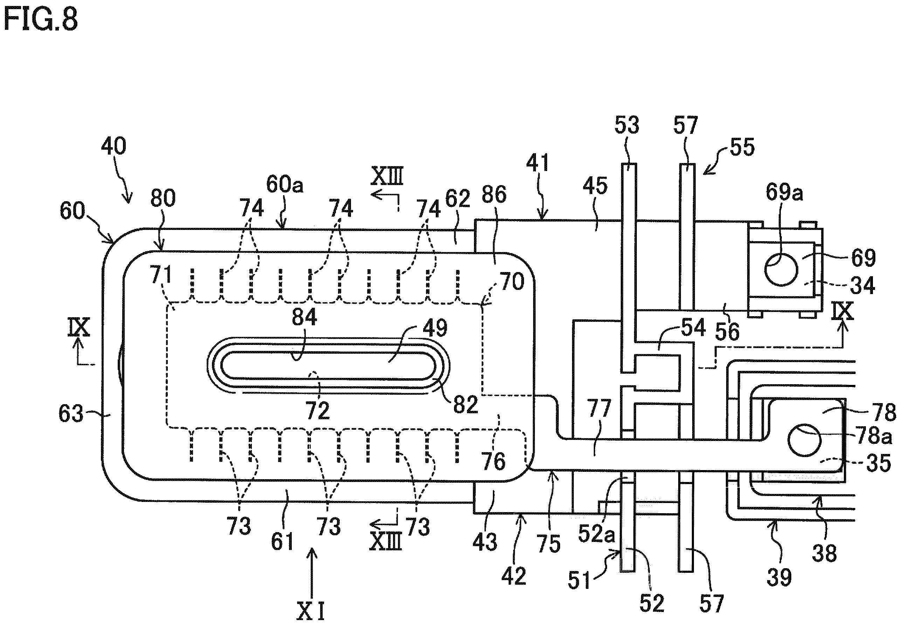

The cylindrical wall portion (81) is in a cylindrical shape having an oblong cross-section elongated in the left-and-right direction. A lower edge portion of the inner periphery of the cylindrical wall portion (81) is provided with an oblong annular projection (82) (see FIG. 5 and FIG. 8). The annular projection (82) projects inward from an inner peripheral surface of the lower edge of the cylindrical wall portion (81). The bottom surface of the cylindrical wall portion (81) and the bottom surface of the annular projection (82) are flush with each other and form a horizontal plane.

An oblong opening (84) elongated in the left-and-right direction is formed by being surrounded by the annular projection (82). The projection (49) of the insulating member (41) is fitted into the oblong opening (84). As a result, the stabilizer (80) is arranged above the electrode support plate (71), and a relative positional relationship among the stabilizer (80), the electrode support plate (71), and the counter electrode (60) is determined. In the state in which the projection (49) is fitted in the oblong opening (84), a gap is left between the projection (49) and the inner peripheral surface of the cylindrical wall portion (81).

The profile of the flanged portion (86) has a rectangular plate-like shape elongated in the left-and-right direction. In the state in which the projection (49) is fitted in the cylindrical wall portion (81), the flanged portion (86) is approximately horizontal. The flanged portion (86) projects forward such that its front edge is positioned forward of the tips of the first discharge needles (73). The flanged portion (86) projects rearward such that its rear edge is positioned rearward of the tips of the second discharge needles (74). In other words, the flanged portion (86) covers the entire portions of the first discharge needles (73) and the second discharge needles (74) from above. The bottom surface of the flanged portion (86) forms a horizontal plane and is parallel with the respective discharge needles (73, 74) along the discharge needles (73, 74).

### -Operation-

Operation of the air conditioner (10) will be described. The air conditioner (10) illustrated in FIG. 1 switches between a cooling operation and a heating operation. When the fan (17) of the air conditioner (10) is actuated, air in the indoor space (S) is drawn into the air passage (11a) through the room-air duct (12). This air passes through the prefilter (14). The prefilter (14) catches relatively-large dust contained in the air.

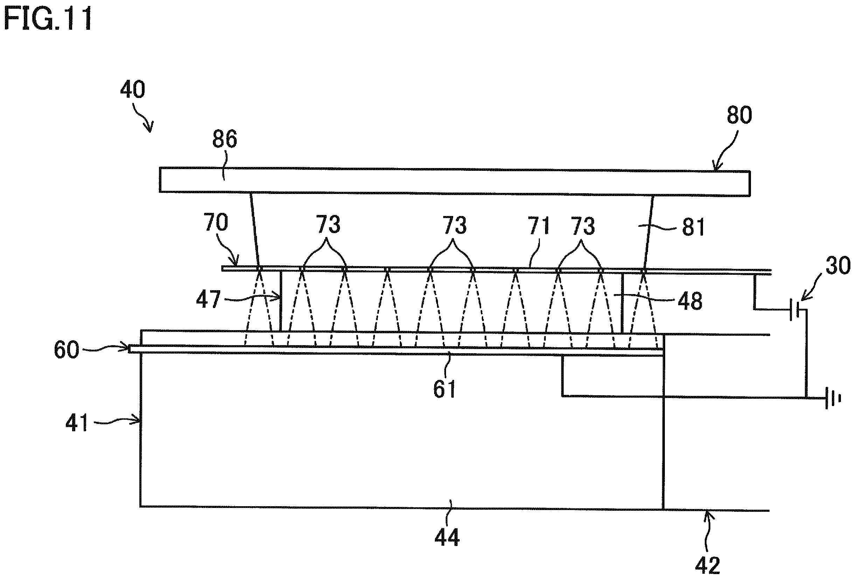

The air which has passed through the prefilter (14) passes through the discharge unit (20) (see FIG. 2). Specifically, the air flows into the processing chamber (27) through the first air hole (28) of the casing (21). In the discharge unit (20), a high voltage is supplied from the power transformer (33) of the voltage supply section (30) to the discharge electrode (70). As a result, a streamer discharge develops from the tip of each discharge needle (73, 74) of the discharge electrode (70) to the counter plates (61, 62) (see FIG. 11). The high voltage is also supplied to the stabilizer (80) connected to the discharge electrode (70). Thus, the streamer discharges emerged from the discharge needles (73, 74) to the counter plates (61, 62) are stabilized.

When a streamer discharge is generated in the discharge process section (40), active species are generated in the air due to the streamer discharge. These active species oxidize and decompose harmful or odor components in the air, thereby purifying the air. The air in the processing chamber (27) flows out of the casing (21) from the second air hole (29) together with the active species (see FIG. 3) and passes through the catalyst filter (15). The catalyst filter (15) adsorbs odor components in the air. The adsorbed odor components are decomposed by the active species, thereby regenerating the adsorbent.

The air purified in this manner is heated or cooled by the heat exchanger (16) and is then supplied into the indoor space (S) through the air-supply duct (13). As a result, the indoor space (S) is heated or cooled, and the indoor air is purified.

### <Fabrication Process of Discharge Unit>

Now, a fabrication process of the discharge unit (20) will be described. The fabrication process includes a process for fabricating the discharge process section (40) and a placement process for mounting the discharge process section (40) and the voltage supply section (30) into the casing (21).

### <Fabrication Process of Discharge Process Section>

The fabrication process of the discharge process section (40) will be described with reference to FIG. 5. In the fabrication process of the discharge process section (40), the insulating member (41), the discharge electrode (70), and the stabilizer (80) are assembled to one another sequentially from bottom to top.

In the first step, the insulating member (41) and the counter electrode (60) are formed as an integrally-formed unit by insert molding.

In the second step, the projection (49) of the insulating member (41) is fitted in the positioning hole (72) of the discharge electrode (70). As a result, the electrode support plate (71) is arranged on the mounting surface (50), thereby achieving positioning and temporary fixing of the discharge electrode (70).

In the third step, the projection (49) of the insulating member (41) is fitted into the cylindrical wall portion (81) of the stabilizer (80). As a result, the cylindrical wall portion (81) of the stabilizer (80) is arranged above the electrode support plate (71). In this state, the electrode support plate (71) is sandwiched between the mounting surface (50) and the cylindrical wall portion (81).

In the fourth step, the projection (49) is fixed to the stabilizer (80) and the electrode support plate (71). Specifically, this fixing is achieved by ultrasonic wave welding. That is, the tip of the projection (49) is melted by an ultrasonic wave, and the melted resin flows into a space above the annular projection (82) and a gap around the oblong opening (84). When the melted resin solidifies, the stabilizer (80) and the projection (49), as well as the projection (49) and the electrode support plate (71), are fixed to each other. As a result, the discharge electrode (70) is fixed or supported between the stabilizer (80) and the insulating member (41).

In the fourth step, the stabilizer (80) and the projection (49) do not necessarily have to be fixed to each other by the ultrasonic wave welding, but may be fixed by other techniques, such as thermal welding, vibration welding, and bonding.

### <Placement Process>

Now, a process for assembling the discharge process section (40) and the voltage supply section (30) into the casing (21) will be described. In this placement process, all the elements are assembled from above the lower case (22).

First, the voltage supply section (30) is assembled to the bottom of the top-open lower case (22) closer to the right (i.e., the bottom of the housing chamber (26)).

Next, the discharge process section (40) built in the above-described manner is assembled to the bottom of the lower case (22) closer to the left (i.e., the bottom of the processing chamber (27)). In this assembly, as illustrated in FIG. 4, the earth connector (69) is connected to the earth terminal (34) with the screw (37), and the feed connecting portion (78) is connected to the feed terminal (35) with the screw (36). It is therefore possible to supply a voltage from the voltage supply section (30) to the discharge electrode (70).

Then, the upper case (23) is assembled on top of the lower case (22). The discharge unit (20) illustrated in FIG. 2 and FIG. 3 is obtained in this manner.

### <Detailed Configuration and Advantages of Insulating Member>

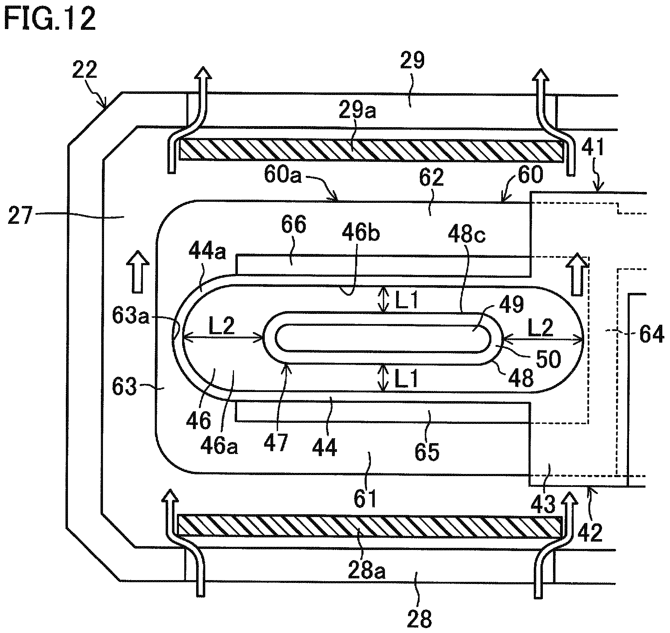

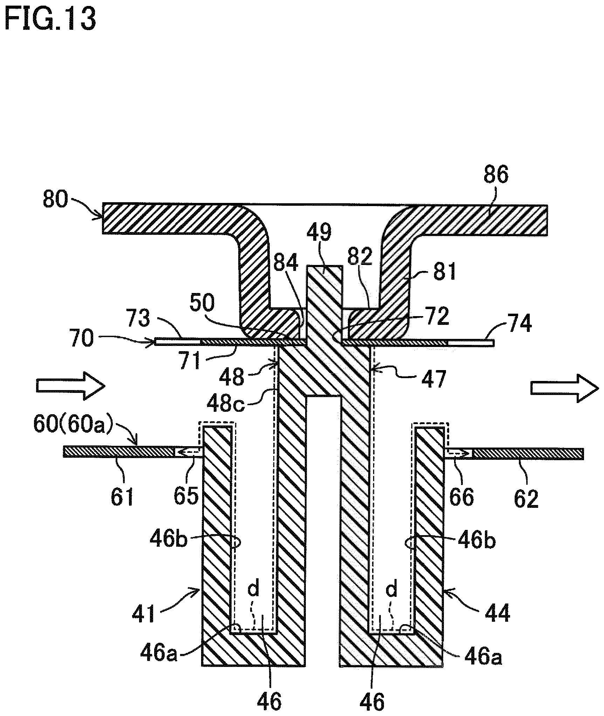

As illustrated in FIG. 12 and FIG. 13, a creepage surface (which is indicated by a broken arrow d in FIG. 13) is formed between the discharge electrode (70) and each counter plate (61, 62). The creepage surface is a continuous surface consisting of an outer wall surface (48c) of the support body (48), a bottom surface (46a) of the oblong groove (46), and an inner wall surface (46b) of the oblong groove (46). The creepage distance between the discharge electrode (70) and each counter plate (61, 62) is therefore increased, and the insulation resistance of the insulating member (41) is maintained.

As illustrated in FIG. 12, the base (44) has a shape elongated in the left-and-right direction. In the insulating member (41), the length L1 and the length L2 are determined to satisfy L1 > L2, where L1 represents a distance between an outside surface of the support body (48) in the width direction and an inside surface of the oblong groove (46) in the width direction, and L2 represents a distance between a longitudinal end on the outside of the support body (48) and a longitudinal end on the inside of the oblong groove (46). This structure may easily result in a reduced distance, and hence the reduced creepage distance, between the discharge electrode (70) and each counter plate (61, 62). However, as described above, the provision of the gaps (65, 66) between the base (44) and each counter plate (61, 62) may ensure a sufficient creepage distance between the base (44) and each counter plate (61, 62).

The oblong groove (46) is a recess formed in the base (44) and is recessed in a direction intersecting with the flow of air in the processing chamber (27). Specifically, the air flows through the processing chamber (27) from the front side to the rear side. On the other hand, the oblong groove (46) is recessed in a downward direction intersecting with the airflow direction. Thus, the amount of air which flows through the processing chamber (27) and comes into the oblong groove (46) may be reduced, which in turn may reduce the amount of dust or other particles in the air adhering to the inner wall of the oblong groove (46). As a result, it is possible to prevent a leakage current from increasing on the inner wall surface of the oblong groove (46) due to the adhesion of the dust or other particles, and maintain the insulation resistance of the insulating member (41).

As illustrated in FIG. 12, the base (44) extends in a direction (i.e., in the left-and-right direction) which is orthogonal to (i.e., intersects with) the flow of air, and the oblong groove (46), too, extends in a direction (i.e., in the left-and-right direction) which is orthogonal (i.e., intersects) with the flow of air. Thus, the distance in which the air in the processing chamber (27) flows above the oblong groove (46) is relatively shortened. This structure may therefore prevent dust or other particles in the air from adhering to the inner wall of the oblong groove (46) with more effectively.

As illustrated in FIG. 12, the first shielding plate (28a) is located on the rear side of the first air hole (28), and the second shielding plate (29a) is located on the rear side of the second air hole (29). The first shielding plate (28a) and the second shielding plate (29a) overlap with the base (44) and the support (47) in the airflow direction (i.e., the direction from the front to rear side). The dust or other particles in the air flowing into the first air hole (28) adheres to the surface of the first shielding plate (28a), as well. Thus, the dust or other particles in the air may be prevented from adhering to the inner wall of the oblong groove (46).

Further, the air coming into the first air hole (28) goes around the outer edges (i.e., outer portions in the left-and-right direction) of the first shielding plate (28a) and flows into the processing chamber (27). Then, the air in the processing chamber (27) flows to the second air hole (29) through the outer edges (i.e., outer portions in the left-and-right direction) of the second shielding plate (29a). Thus, in the processing chamber (27), the amount of air flowing through the outer portions, in the longitudinal direction, of the base (44) is relatively large (see the open arrows in FIG 12). The dust or other particles in the air may thus be prevented from adhering to the outer wall surface (48c) of the support body (48), which may prevent a reduction in the insulation resistance of the insulating member (41) more effectively.

### -Advantages of Embodiment-

According to the above embodiment, the oblong groove (46) formed in the insulating member (41) is recessed in the direction intersecting with the airflow. Thus, the dust in the air may be prevented from entering into the oblong groove (46). As a result, it is possible to prevent the dust from adhering to the inner wall surface (46b) of the oblong groove (46) and thus prevent reduction in the insulation resistance of the insulating member (41). This may ensure a sufficient potential difference between the discharge electrode (70) and the counter electrode (60) and achieve desired streamer discharges.

The support (47) is located at a middle portion, in the left-and-right direction and the front-and-rear direction, of the oblong groove (46). This structure allows for a uniform creepage distance from the support (47) to the inner wall surface (46b) of the oblong groove (46), which may avoid a situation in which part of the creepage distance is significantly shortened and a leakage current increases. As a result, a sufficient insulation resistance of the insulating member (41) may be ensured.

The base (44) and the oblong groove (46) extend in the left-and-right direction orthogonal to the airflow. Thus, the distance in which the air flows above the oblong groove (46) may be minimized. As a result, it is possible to further reduce the amount of dust in the air which adheres to the inner wall surface (46b) of the oblong groove (46) or other surfaces.

The provision of the gaps (65, 66) between the base (44) and each counter plate (61, 62) may further increase the creepage distance between the discharge electrode (70) and each counter plate (61, 62). In addition, the electrode support plate (71) is configured to be able to support a large number of discharge needles (73, 74) on both side edge portions in the width direction of the electrode support plate (71), which may contribute to a further increase in the amount of active species generated by a discharge.

### <<Other Embodiments>>

The above embodiment may also have the following structures.

The recess (46) formed in the base (44) is configured as the oblong groove (46) elongated in the left-and-right direction, but this is a non-limiting example. The transverse cross-sectional view of the recess (46) may have a rectangular, circular, oval or any other shapes.

The air conditioner (10) of the above-described embodiment is installed on the back side of the ceiling (C). However, the discharge unit (20) of the present invention may be applied to an air conditioner of any other types, such as a wall-hung type, a ceiling-embedded type, and a ceiling-suspended type. The discharge unit (20) according to the present embodiment may be applied to an air cleaner.

### INDUSTRIAL APPLICABILITY

As can be seen from the foregoing description, the present invention is useful as a discharge unit.

### DESCRIPTION OF REFERENCE CHARACTERS

- 20: Discharge Unit
- 21: Casing
- 30: Voltage Supply Section
- 41: Insulating Member
- 44: Base
- 46: Oblong Groove (Recess)
- 47: Support
- 60: Counter Electrode
- 70: Discharge Electrode
- 71: Electrode Support Plate
- 73: First Discharge Needle (Discharge Needle)
- 74: Second Discharge Needle (Discharge Needle)

## Claims

1. A discharge unit comprising:
a casing (21) having a front side opposite of a rear side, a top side opposite of a bottom side and two opposing lateral sides, in which an air passage (27) extending from the front side of the casing (21) to the rear side of the casing (21) is formed to allow air to pass therethrough;
a discharge electrode (70) and a counter electrode (60) located in the air passage (27); and
a voltage supply section (30) configured to give a potential difference between the discharge electrode (70) and the counter electrode (60), wherein
the discharge unit includes an insulating member (41) having a support (47) which supports the discharge electrode (70) and a base (44) which supports the counter electrode (60),
the counter electrode (60) is supported on a periphery of the base (44),
the base (44) has a recess (46) recessed in a direction extending from the top side of the casing (21) to the bottom side of the casing (21) and intersecting with a flow of air flowing through the air passage (27), and
the support (47) extends from a bottom of the recess (46) to protrude outward from an open end of the recess (46),
wherein the base (44) has an elongated shape extending in a direction extending from a lateral side of the casing (21) to the other lateral side of the casing (21) and intersecting with the flow of air, and
the recess (46) has an elongated shape extending in the direction extending from a lateral side of the casing (21) to the other lateral side of the casing (21) along the base (44).

2. The discharge unit of claim 1, wherein
the support (47) is located at a central position of the recess (46).

3. The discharge unit of claim 1, wherein
the discharge electrode (70) includes an electrode support plate (71) extending longitudinally in a direction extending from a lateral side of the casing (21) to the other lateral side of the casing (21) along the base (44) and supported by the support (47), and elongated discharge needles (73, 74) arranged at longitudinal side edges of the electrode support plate (71) and protruding from the longitudinal side edges,
the counter electrode (60) includes counter plates (61, 62) which extend along a longitudinal direction of the base (44) and to which tips of the discharge needles (73, 74) are opposed, and
gaps (65, 66) extending along the longitudinal direction of the base (44) are formed between the base (44) and the counter plates (61, 62).

## Patentansprüche

1. Entladungseinheit, umfassend:
ein Gehäuse (21) mit einer Vorderseite gegenüber einer Rückseite, einer Oberseite gegenüber einer Unterseite und zwei gegenüberliegenden lateralen Seiten, in dem ein Luftkanal (27), der sich von der Vorderseite des Gehäuses (21) zur Rückseite des Gehäuses (21) erstreckt, ausgebildet ist, um den Durchgang von Luft zu ermöglichen;
eine Entladungselektrode (70) und eine Gegenelektrode (60), die in dem Luftkanal (27) angeordnet sind; und
einen Spannungsversorgungsabschnitt (30), der so konfiguriert ist, dass er eine Potentialdifferenz zwischen der Entladungselektrode (70) und der Gegenelektrode (60) erzeugt, wobei
die Entladungseinheit ein isolierendes Element (41) mit einem Träger (47), der die Entladungselektrode (70) trägt, und einer Basis (44), die die Gegenelektrode (60) trägt, umfasst,
die Gegenelektrode (60) auf einem Umfang der Basis (44) getragen wird,
die Basis (44) eine Ausnehmung (46) aufweist, die in einer Richtung vertieft ist, die sich von der Oberseite des Gehäuses (21) zur Unterseite des Gehäuses (21) erstreckt und einen durch den Luftkanal (27) strömenden Luftstrom schneidet, und
sich der Träger (47) von einem Boden der Ausnehmung (46) erstreckt, um von einem offenen Ende der Ausnehmung (46) nach außen vorzustehen,
wobei die Basis (44) eine längliche Form hat, die sich in einer Richtung erstreckt, die sich von einer lateralen Seite des Gehäuses (21) zu der anderen lateralen Seite des Gehäuses (21) erstreckt und den Luftstrom schneidet, und
die Ausnehmung (46) eine längliche Form hat, die sich in der Richtung erstreckt, die sich von einer lateralen Seite des Gehäuses (21) zu der anderen lateralen Seite des Gehäuses (21) entlang der Basis (44) erstreckt.

2. Entladungseinheit nach Anspruch 1, wobei
der Träger (47) in einer zentralen Position der Ausnehmung (46) angeordnet ist.

3. Entladungseinheit nach Anspruch 1, wobei
die Entladungselektrode (70) eine Elektrodentragplatte (71), die sich longitudinal in einer Richtung, die sich von einer lateralen Seite des Gehäuses (21) zu anderen lateralen Seite des Gehäuses (21) erstreckt, entlang der Basis (44) erstreckt und von dem Träger (47) getragen wird, und längliche Entladungsnadeln (73, 74) aufweist, die an den Längsseitenrändern der Elektrodentragplatte (71) angeordnet sind und von den Längsseitenrändern vorstehen,
die Gegenelektrode (60) Gegenplatten (61, 62) aufweist, die sich entlang einer Längsrichtung der Basis (44) erstrecken und denen Spitzen der Entladungsnadeln (73, 74) gegenüberliegen, und
zwischen der Basis (44) und den Gegenplatten (61, 62) Spalte (65, 66) gebildet sind, die sich entlang der Längsrichtung der Basis (44) erstrecken.

## Revendications

1. Unité de décharge comportant :
un boîtier (21) ayant un côté avant à l'opposé d'un côté arrière, un côté de dessus à l'opposé d'un côté de dessous et deux côtés latéraux opposés, dans laquelle un passage d'air (27) s'étendant depuis le côté avant du boîtier (21) jusqu'au côté arrière du boîtier (21) est formé pour permettre à l'air de passe au travers de celui-ci ;
une électrode de décharge (70) et une contre-électrode (60) se trouvant dans le passage d'air (27) ; et
une section d'alimentation en tension (30) configurée pour donner une différence de potentiel entre l'électrode de décharge (70) et la contre-électrode (60), dans laquelle
l'unité de décharge comprend un élément d'isolation (41) ayant un support (47) qui supporte l'électrode de décharge (70) et une base (44) qui supporte la contre-électrode (60),
la contre-électrode (60) est supportée sur une périphérie de la base (44),
la base (44) a une partie en retrait (46) mise en retrait dans une direction s'étendant depuis le côté de dessus du boîtier (21) jusqu'au côté de dessous du boîtier (21) et croisant un écoulement d'air s'écoulant au travers du passage d'air (27), et
le support (47) s'étend depuis un fond de la partie en retrait (46) pour faire saillie vers l'extérieur depuis une extrémité ouverte de la partie en retrait (46),
dans laquelle la base (44) a une forme allongée s'étendant dans une direction s'étendant depuis un côté latéral du boîtier (21) jusqu'à l'autre côté latéral du boîtier (21) et croisant l'écoulement d'air, et
la partie en retrait (46) a une forme allongée s'étendant dans la direction s'étendant depuis un côté latéral du boîtier (21) jusqu'à l'autre côté latéral du boîtier (21) le long de la base (44).

2. Unité de décharge selon la revendication 1, dans laquelle
le support (47) est situé au niveau d'une position centrale de la partie en retrait (46).

3. Unité de décharge selon la revendication 1, dans laquelle
l'électrode de décharge (70) comprend une plaque de support d'électrode (71) s'étendant dans le sens longitudinal dans une direction s'étendant depuis un côté latéral du boîtier (21) jusqu'à l'autre côté latéral du boîtier (21) le long de la base (44) et supportée par le support (47), et des aiguilles de décharge allongées (73, 74) agencées au niveau de bords latéraux longitudinaux de la plaque de support d'électrode (71) et faisant saillie depuis les bords latéraux longitudinaux,
la contre-électrode (60) comprend des contre-plaques (61, 62) qui s'étendent le long d'une direction longitudinale de la base (44) et auxquelles des pointes des aiguilles de décharge (73, 74) sont opposées, et
des espaces (65, 66) s'étendant le long de la direction longitudinale de la base (44) sont formés entre la base (44) et les contre-plaques (61, 62).
